# EUROPEAN PATENT APPLICATION

(11) **EP 3 375 417 A1**
(43) Date of publication of application: **19.09.2018**
(21) Application number: 17305280.4
(22) Date of filing: 15.03.2017
(51) Int. Cl.: A61F 9/00, A61N 1/05, A61N 1/36

(54) **ACTIVE LENS BASED ON GAN SEMICONDUCTOR OPTICAL AMPLIFIER**

(71) Applicant: Nokia Solutions and Networks Oy, 02610 Espoo (FI)
(72) Inventor: GARREAU, Alexandre, 91767 PALAISEAU (FR); BRENOT, Romain, 91767 PALAISEAU (FR); AUBRY, Raphaël, 91767 PALAISEAU (FR)
(74) Representative: Novagraaf Technologies

(57) **Abstract**

The present invention relates to devices for amplifying the light incoming on the retina.

The device comprises optical amplifier means adapted to amplify visible light incoming on the retina. Preferably, the optical amplifier means include an array of semiconductors composed of elements which are transparent at visible wavelengths. This device can be integrated into an active crystalline lens prosthesis or a corrective glass.

The invention addresses the major drawbacks of the current apparatuses that treat person suffering retinal degenerative conditions. It is easy to manufacture, easy and safe to implement, and low cost.

## Description

### SPECIFICATION

### Field of the Invention

The present invention relates to method and device for amplifying light incoming on a retina.

### Background

The retina is an inner coat of the eye which is a light-sensitive layer of tissue. Photoreceptors are light-sensing cells of the retina, while other cells, like the retinal ganglion cells process photoreceptor signals and send information to the brain via the optic nerve. There are two types of photoreceptors: cones and rods. The entire retina contains about 7 million cones, functioning mainly in daylight and for perception of color, and 75 to 150 million rods, functioning mainly in dim light and for black and white vision).

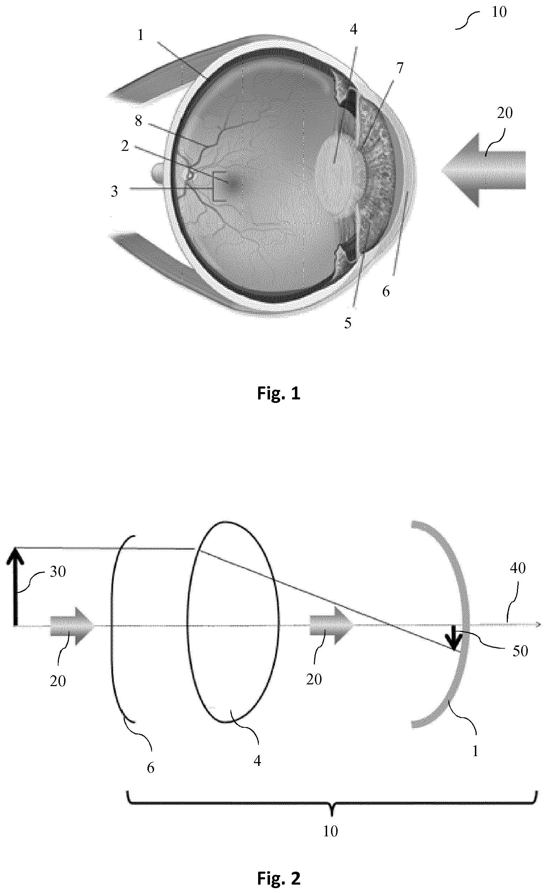

As shown in **Figure 1****,** the retina 1 includes different regions, including the foveola which is located within a region called the macula 3 (6mm in diameter), a yellow section of the human retina. The foveola is approximately 0.35mm in diameter and lies in the center of the fovea 2 (1.5mm in diameter) and contains only cone cells. In this region the cone receptors are found to be longer, slimmer and more densely packed than anywhere else in the retina. It allows that region to have the highest visual acuity.

When photoreceptors degenerate due to disease like retinitis pigmentosa or age-related macular degeneration, the retina can no longer respond to light. However, the other nerve cells of the retina remain in sufficient numbers that electrical stimulation of these cells results in the perception of light.

Currently two approaches are studied to compensate this acuity loss and treat persons who suffer retinal degenerative conditions: one based on retinal prostheses, the other one based on optogenetics.

### Retinal prostheses

Retinal prostheses consist of an array of electrodes that are activated, either by light entering the eye in the case of a subretinal prosthesis or by a signal from a camera mounted outside the eye in the case of an epiretinal prosthesis.

The epiretinal approach involves sticking an electronic implant in front of the retina to stimulate the nerve cells. The surgery to do this is rather straightforward, but the epiretinal implant is not itself light sensitive and needs to be connected to a camera outside the eye. It requires an "encoder", whose function is to take over the role of the neurons of the inner retina, which carry out preliminary processing of visual information. Models have been developed for predicting visual sensitivity of patients with epiretinal prosthesis. Different technologies can be used, usually based on silicon devices. Argus II is the reference epiretinal prosthesis with 60 electrodes manufactured by Second Sight Medical Products Inc. It has been already tested on patients all over the world and has been approved by the United States FDA on 14 February 2013.

The subretinal prosthesis replaces the photoreceptors and is placed underneath the retina, which is technically challenging but stimulates the nerve cells in a more natural position. It converts incident light into an electrical signal which is transmitted on to the bipolar cells. Retina is the reference subretinal prosthesis manufactured by Retina Implant AG. It is itself light sensitive, with a 1500 pixel array and does not need an external camera.

### Optogenetics

The optogenetic technique modifies individual neurons to incorporate light-sensitive protein (Channelrhodopsin-2 or halorhodopsin) into the cell membrane. By making each cell light sensitive, vision can potentially be restored to near-normal acuity. However, artificial vision based on optogenetics has its own challenge: modified cells require bright, blue light (460 nm) to be activated, roughly 7 orders of magnitude above the light sensitivity threshold in normally sighted people.

### Summary

The present invention proposes a device to address the needs of an easy to manufacture, easy and safe to implement, and low cost device, and solve the prior art drawbacks.

In a first and general embodiment, a device for amplifying the light incoming on a retina comprises optical amplifier means adapted to amplify visible light incoming on the retina. Such device improves the vision of somebody having a photoreceptor degeneration, the person could have or not retinal prostheses or could have received or not an optogenetic therapy.

The optical amplifier means can include an array of semiconductors and these semiconductors can be composed of elements which are transparent at visible wavelengths. The transparency of the elements enable to stimulate the normal retinal cells which is not possible in presence of conventional epiretinal prosthesis implant in front of the retina.

To create a biocompatible device, the semiconductors may have active layers encapsulated in a biocompatible material.

Advantageoulsy, the active layers form a multi quantum wells structure or layers composed of quantum dots or quantum dashes, electrically separated from each other, so as to prevent electrical interactions / interferences between the different quantum wells.

To power the optical amplifiers and/or to polarize the device, the semiconductor may have a GaN substrate and top layer forming a p-n junction, the substrate and the top layer having opposite doping.

So as to protect the device from environment, the semiconductor diode can be insulated by an insulating layer.

Advantageously, to compensate a focusing problem of somebody having a photoreceptor degeneration, the device can further comprise a first microlens array at an entry of the semiconductor optical amplifiers array and adapted to focus the image of an object on the retina.

To compensate the divergence sometimes generated by the optical amplifiers, the device could further comprise a second microlens array at an output of the semiconductors array and adapted to compensate a divergence of the amplified light beam at the output of the semiconductor optical amplifiers array.

Advantageously, the device can further comprise an optical coating adapted to suppress the amplified spontaneous emission generated in the optical amplifiers and avoiding the generation of a laser. Such amplified spontaneous emission could damage the eye or be dangerous to the environment if not controlled. The optical coating could be an antireflective coating.

In order to improve the visual acuity of somebody having a photoreceptor degeneration, it could be advantageous to optimize the light received at the maximum of the human eye detection. So the optical amplifier means could be adapted to specifically amplify light having a wavelength comprised within a predetermined range.

To activate light-sensitive proteins used in optogenetic therapy, the optical amplifier means may be adapted to stimulate light-sensitive proteins incorporated in the retina area by an optogenetic technique.

The present invention also relates to an active crystalline lens prosthesis comprising a device as described above. The crystalline lens being more accessible than the retina and offering more space, such prosthesis minimizes congestion and simplifyies technology.

The present invention further relates to a corrective glass comprising a device as described above. Such corrective glass enables to avoid a complex surgery to heal patients suffering Age-related Macular Degeneration.

Finally, the present invention also relates to a method for amplifying the light incoming on a retina to improve the vision of somebody having a photoreceptor degeneration. The method comprises two steps:
- placing a device, as described above, in front of the retina of a user, so that the light incoming on the retina is amplified by the optical amplifier means; and
- adjusting the gain of the optical amplifier means.

Advantageously, the method further comprises a step of configuring the device to form a clear image of a distant scene on the retina, to improve the vision of somebody having also focusing problem.

### Brief Description of the Drawings

Some embodiments of apparatus in accordance with embodiments of the present invention are now described, by way of example only, and with reference to the accompanying drawings, in which :
- Figure 1 schematically illustrates the anatomy of a human eye.
- Figure 2 schematically illustrates the optical diagram of an eye.
- Figure 3 schematically illustrates a vertical design according to a first device embodiment of the invention.
- Figures 4-5 schematically illustrate side views of an horizontal design according to a second device embodiment;
- Figures 6 schematically illustrates a third device embodiment;
- Figure 7 schematically illustrates a single semiconductor optical amplifier element according to a device embodiment;
- Figures 8 and 9 schematically illustrate a single semiconductor optical amplifier element according to two different device embodiment having two ways to integrate optical coatings;
- Figure 10 schematically illustrates an active crystalline lens prosthesis embodiment;
- Figure 11 schematically illustrates a corrective glass embodiment; and
- Figure 12 schematically illustrates a flowchart of a method for amplifying the light incoming on a retina according to an embodiment of the invention.

### Description of Embodiments

As described before, **Figure 1** illustrates the anatomy of a human eye 10, and **Figure 2** illustrates the normal way such a human eye 10 optically operates.

An object 30 is observed at the infinity, located in a plan perpendicular to the optical axis 40. The light 20 coming from the object 30 passes through the cornea 6, which can be assimilated as a transparent refracting surface which does not modify the light 20 (no intensity change, no optical deviation). Then, the lens 4 projects the image of the object 30, and thus the light 20 coming from it, onto the retina 1.

As described before, there are two main types of eye degeneration:
- photoreceptors degeneration, when the light sensitivity of the retina decreases, and
- cataract, when the lens 4 becomes cloudy and decreases the intensity of the light received by the retina.

To restore or improve the vision, the present invention proposes a device for amplifying the light 20 incoming on the retina 1 comprising optical amplifier means, adapted to amplify visible light incoming on the retina.

In a general device embodiment, the optical amplifier means include an array of semiconductors which are easy to manufacture.

Advantageously, the semiconductors are composed of elements which are transparent at visible wavelengths such as Gallium nitride (GaN) semiconductors.

In fact, GaN semiconductors present the interest to have a good chemical stability and to be biocompatible. They are remarkable in that they are transparent in the visible range. In such an embodiment, the normal retinal cells are still stimulated because they are not masked.

GaN is a binary III/V direct bandgap semiconductor commonly used in light-emitting diodes since the 1990's. Because of its hardness, mechanical stability, its high heat capacity and thermal conductivity, GaN is wildly used in military and space applications, but not in the ophthalmic field.

Usually, GaN devices are used in ultraviolet range: bulk GaN nanowire photodetectors working in photovoltaic or photoconductive operation modes. Indium Gallium Nitride (InGaN) / GaN multi quantum wells are also well known for blue laser. Moreover, nitride-based photodiodes at 510 nm wavelength have been realized recently.

Advantageously, the semiconductors include active layers comprising several epitaxies of InGaN and Aluminium Gallium Nitride (AlGaN) or InGaN and Gallium Arsenic Nitride (GaAsN) so that, the InGaN/AlGaN or InGaN/GaAsN multi-quantum wells (or layers composed of quantum dots or quantum dashes) form a transparent array of optical amplifiers at visible wavelength (the visible wavelength generally ranges between 400nm to 800nm).

Advantageously, the different quantum wells / dots / dashes of the array are electrically separated to prevent electrical interactions or interferences. This isolation can be realised with an insulation layer 150 or by implantation of hydrogen ions H+ or by etching to reach the insulated substrate or layer.

GaN being also biocompatible, the active layers of the semiconductors can be encapsulated between a GaN substrate and a GaN layer grown to finish the epitaxy.

It is to be noted that other biocompatible and transparent materials could be used for the encapsulation such as carbon, diamond, titanium dioxide or silicon.

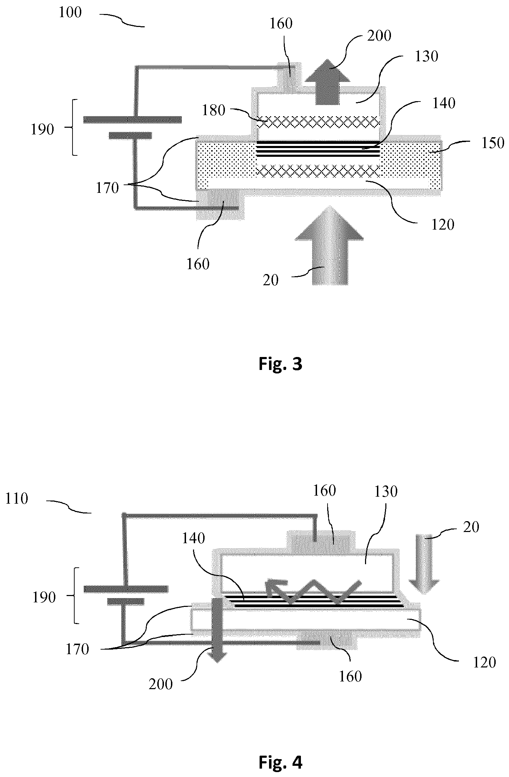

**Figure 3** illustrates an embodiment of the device according to a vertical design 100.

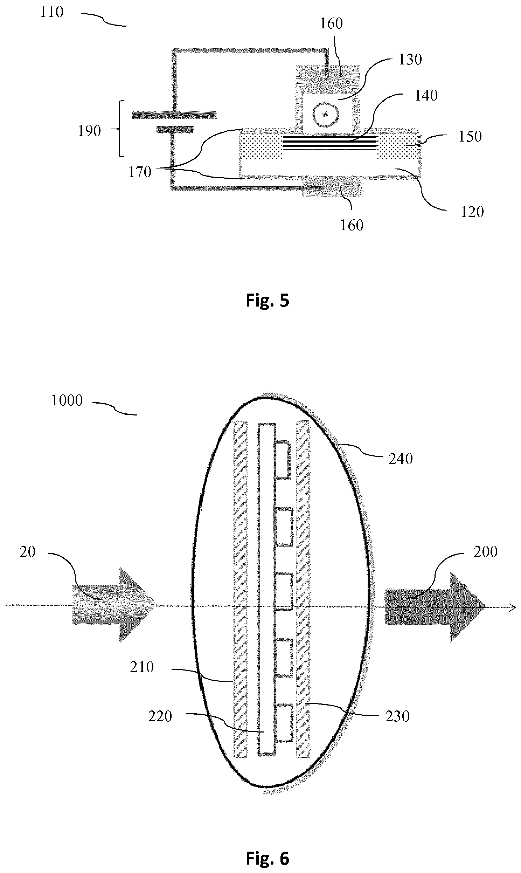

**Figures 4** **and** **5** illustrate side views of another embodiment of the device according to a horizontal design 110.

The global design is common for both optoelectronic devices 100 and 110. It is based on a structure formed by one or several epitaxies of InGaN/GaNAs or InGaN/AlGaN multi-quantum wells 140 as intrinsic active layer on a GaN Substrate 120. It is possible to define a structure including several multi quantum wells 140, by using Selective Area Growth technology (SAG), in order to obtain one or several amplified or detected wavelengths (blue, green, yellow).

On top of the structure, a top epitaxied GaN layer 130 is grown to finish the epitaxy. If the GaN substrate 120 is doped p, the top epitaxied layer 130 is doped n. Reciprocally, if the GaN substrate 120 is doped n, the top epitaxied layer 130 is doped p, so that the substrate and the top layer form a p-n junction. In order to power the optical amplifiers, the two p and n doping must be implemented and connected to electrical contacts 160 linked to a power source 190.

Different optical amplifier quantum wells belonging to a same array are electrically separated from each other by an insulation 150 which can be implanted GaN or semi-insulating GaN.

The device can also be isolated by an insulating layer 170 that must be a dielectric material or a semi-insulating semiconductor. As specified above, it must have a good biocompatibility like carbon like diamond, Titanium dioxide, common dielectric materials (silica, silicon nitride...) or semi-insulating GaN, and be transparent.

The encapsulation of the active layers by a biocompatible material enables to use hazardous materials to compose the active layers, so that the device will still be well received by the organism.

To form an optical cavity enabling the amplification of the light, the quantum well amplified region 140 can be put between two Distributed Bragg Reflector 180, without working as a cavity surface emitting laser.

In the vertical design 100 illustrated **Figure 3****,** the light 20 coming from the object passes through the substrate 120, a first Distributed Bragg Reflector 180. The light 20 is then amplified by the quantum well amplified region 140 after one (or more) round-trip in the optical cavity formed by the Distributed Bragg Reflectors 180 before exiting through the top layer 130 and being send to the retina 6.

The dimension of the device is from 5µm to 100µm. Because the light 20 enters, in this example, through the top layer 130 to reach the amplifying region 140, the metal contact 160 must not cover the entire top layer 130 surface.

**Figures 4** **and** **5** illustrate side views of the device 110 in which, the active layers 140 and the top layer 130 are not completely aligned. In fact, the quantum well amplified region 140 has two bevelled edges. One of the bevelled edges is the entrance of light 20. The second one is the output of amplified light 200. The bevelled edges are inclined with the Brewster's angle to avoid beam reflexion. The light 20 propagates in the plan of the Figure 4, and the substrate 120 is etched so as to let the light pass through.

**Figure 6** illustrates the device 1000, which, according to an option of the invention, further comprises a first microlens array 210 at the entry of the semiconductor optical amplifiers array 220. This first microlens array 210 is adapted to focus the image of an object 30 on the retina 1 in case the lens 4 does not project correctly the image of the object 40 on the retina. This can happen when the person suffer myopia of hyperopia for example.

According to another option, the device 1000 further comprises a second microlens array 230 at the output of the semiconductor optical amplifiers array 220. This second microlens array 230 is adapted to compensate a divergence of the amplified light beam at the output of the semiconductor optical amplifiers array 220.

Advantageously, the device 1000 further comprises an optical coating 240 adapted to suppress the amplified spontaneous emission (ASE) usually generated in optical amplifiers and avoiding the generation of a laser which could damage the retina 1. Preferably, this optical coating 240 is an antireflective coating.

In **Figure 6****,** the device1000 only comprises one optical coating 240 which protects the retina 4 from damages caused by ASE. Preferably, the device further comprises another optical coating 240 to prevent the device from emitting ASE in the direction of the object, that is to say from the side where the light 20 incomes.

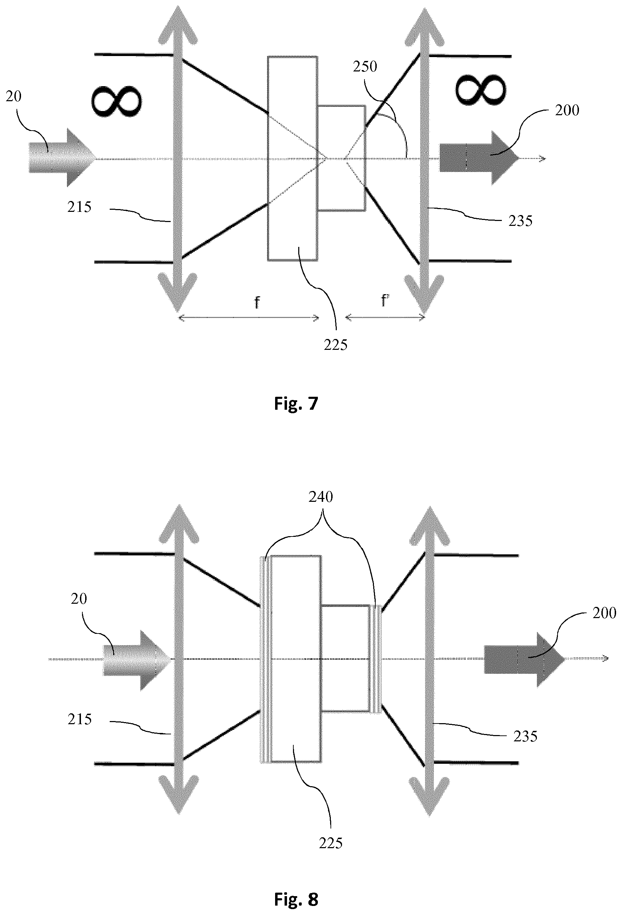

**Figure 7** represents a single optical semi-conductor amplifier 225 of the semiconductors array, located between a first microlens element 215 and a second microlens element 235. At the output of the semiconductor optical amplifier 225, the amplified beam presents a divergence usually associated the waist 250. It is important to collimate the amplified beam to avoid interference between the different beams from other semiconductor optical amplifiers. Hence, in this embodiment of the invention, the second microlens element 235 suppresses this divergence and is located at a focal distance of the output of the semiconductor optical amplifier 225, so that the first 215 and second 235 microlens elements operate as an afocal system ("infinite/infinite": the image of an object located at the infinite is projected at the infinite). The first 215 and second 235 microlens elements can be liquid lenses or solid lenses. The first microlens elements 215 have a focal distance f and the second microlens elements 235 have a focal distance f.

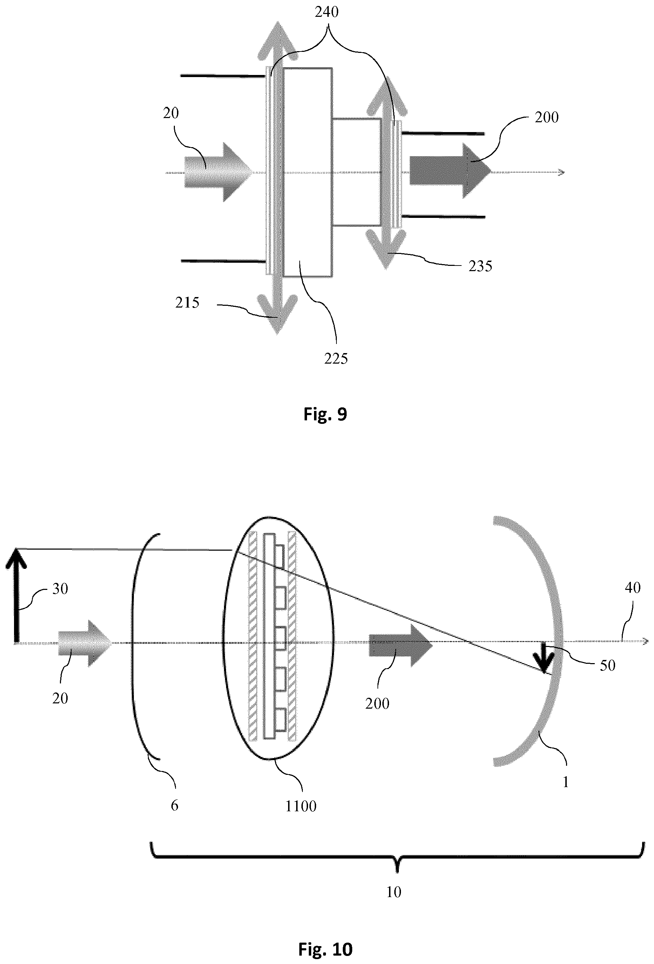

**Figures 8** **and** **9** illustrate two different ways to integrate these optical coatings 240. In **Figure 8****,** the coating is located at the input and output of the semiconductor optical amplifier 225. In **Figure 9****,** a first optical coating 240 is located at the input of the first microlens element 215, and a second optical coating 240 is located at the output of the second microlens element 235. These two configurations can be applied to the input / output of semiconductor optical amplifiers array 220 or at the input of the first microlens array 210 and the output of the second microlens array 230.

In a case where the person, suffering retinal degenerative conditions, would receive an optogenetic treatment with the incorporation of light-sensitive proteins in retina cells activated by a specified wavelength range, there is no need to amplify all the visible light range. So, according to an option of the device, the optical amplifier means can be adapted to specifically amplify a specified wavelength range, preferably the wavelength range that stimulates the light-sensitive proteins. This can be made by adapting the composition of the active layers, so that they amplify only the specified wavelength range and/or by using an optical coating 240 adapted to filter the specified wavelength range and let it pass through the device after being amplified. By doing such things, it is also possible to adjust the wavelength optical amplification in order to obtain the maximum of the human eye detection with or without optogenetics.

In order to prevent any damage to the retina, the gain of the SOA can be limitated so that the power of the amplified signal incoming on the retina is lower than a critical power which could burn retina cells, or generate other damage.

According to another option compatible with the embodiment previously described, the device can further comprise a detector or a probe adapted to measure the ambient brightness of the scene so that the gain of the SOA is adjusted depending on the ambient brightness measure. This will avoid a glare problem to the user on a very sunny day or if the ambient brightness varies quickly, for example.

**Figure 10** represents an active crystalline lens prosthesis 1100 comprising a device as previously described. Implanting an artificial crystalline lens like for cataract surgery is well known compared to implanting a subretinal or an epiretinal prosthesis. Furthermore, the crystalline lens offers more space than a retinal prosthesis which removes technical constraints to manufacture such apparatus and thus reduces associated costs. This active crystalline lens prosthesis 1100 also reduces the surgical difficulties, the lens 4 being more easily accessible than the retina 1.

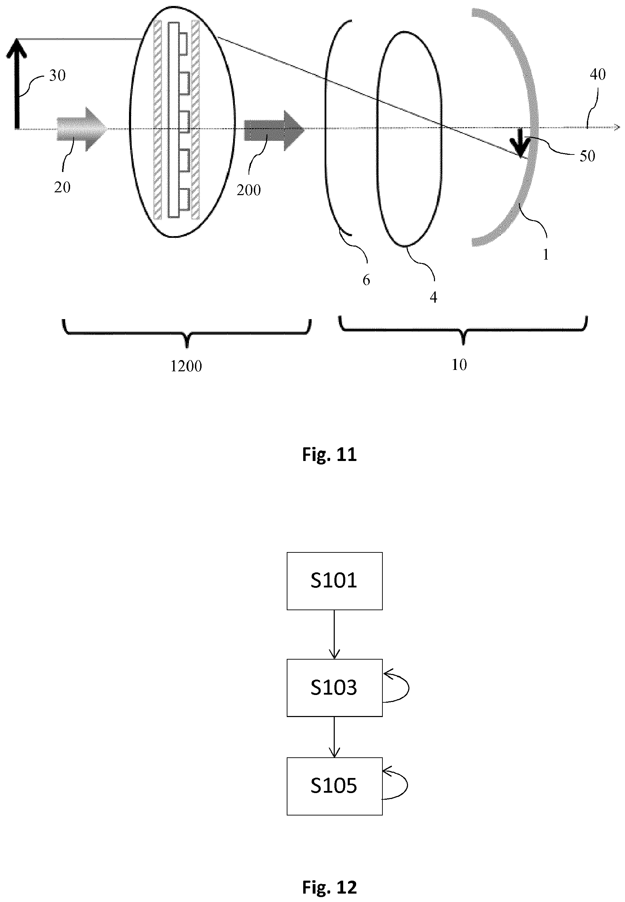

**Figure 11** represents a corrective glass 1200 comprising a device as one previously described. This apparatus enables to suppress surgery. With a device adapted to amplify the visible light a person suffering from retinal degenerative conditions could have improved vision without any surgery. In case, this person would receive an optogenetic treatment, the device used could be adapted to amplify the wavelength range that stimulates the light-sensitive proteins incorporated into the retina cells.

The present invention also relates to a method for amplifying the light incoming on a retina by using one the devices, lens or corrective glasses previously described.

**Figure 12** represents a flowchart of the method according to an embodiment of the present invention.

Step S101:According to a general process embodiment, a device, according to one previously described, is placed in front of the retina of a user so that the light incoming on the retina is amplified by the optical amplifier means. The device can be integrated into an active crystalline lens prosthesis put in the place of the user's lens 4 or into a corrective glass as previously described.

Then, step S103, the gain of the optical amplifier means is adapted. This adjustment depends on:
- which retinal degenerative condition the user suffers and the intensity of the degeneration (for example, if it is cataract and just a decrease in the light intensity received by the eye).
- the user and his or her feelings, like when the ophthalmologist asks, during consultation, whether the correction applied is good or not, based on a series of images that the user looks while the ophthalmologist adjusts the correction for myopia, hyperopia or astigmatism.

For example, the voltage applied to the device may be increased or decreased to vary the gain in function.

Advantageously, the method further comprises a step S105 of configuring the device to form a clear image of a distant scene on the retina.

Configuring may consist in:
- adjusting the wavelength range amplified by the optical amplifier means (for example, as described before, by adapting the active layers of the device or by adapting the optical coating), but also
- adjusting the focal of the first and / or second microlens array (for example, if the microlenses are liquid lenses by varying the voltages applied to the liquid lenses).

This additional step can be applied before or after placing the device in front of the retina of a user depending on:
- whether the device is easily accessible (if integrated into a corrective glass for example) or not (if integrated into an optical crystalline lens prosthesis);
- the defects to corrected and their magnitude (for example myopia, hyperopia or astigmatism, or the correction of glasses of the user);
- whether the user received an optogenetic treatment or not;
- the maximum of the eye detection of the user.

Advantageously, the steps of adjusting the gain S103 and configuring the device S105 can be reiterated to obtain the maximum detection or the most comfortable setting for the user. In particular, when the optical corrections applied are very important compared to the previous ones, they can cause migraines as long as the brain adapts to the new correction. Therefore, it is sometimes preferable to apply a progressive correction than correcting all the defects of the eye at once.

Furthermore, the step of ajusting of the gain of the optical amplifier means S103, like current intensity, can be realized automatically in function of the light environment measured by a detector or a probe.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

### REFERENCES NUMERALS

1 Retina
2 Fovea
3 Macula
4 Lens (Crystalline)
5 Iris
6 Cornea
7 Pupil
8 Blood vessels
10 Human eye
20 light coming from an object and incoming on the eye 10
30 Object
40 Optical axis
50 Image of the object projected on the retina 1
100 Device according to a vertical embodiment
110 Device according to a horizontal embodiment
120 Substrate
130 Top layer
140 Quantum well amplified region / active layers
150 Insulation
160 Electrical contact
170 Insulation layer
180 Distributed Bragg Reflector
190 Power source
200 Light Amplified by the device
210 First microlens array
215 First microlens element
220 Semiconductor optical amplifiers array
225 Semiconductor optical amplifier
230 Second microlens array
235 Second microlens element
240 Optical coating
250 Waist
1000 Device according to another embodiment
1100 Active crystalline lens prosthesis
1200 Corrective glass

## Claims

1. A device for amplifying the light incoming on a retina comprising optical amplifier means adapted to amplify visible light incoming on the retina.

2. The device according to claim 1 wherein the optical amplifier means include an array of semiconductors.

3. The device according to claim 2 wherein the semiconductors are composed of elements which are transparent at visible wavelengths.

4. The device according to claim 2 or 3 wherein the semiconductors have active layers encapsulated in a biocompatible material.

5. The device according to claim 4 wherein the active layers formed a multi quantum wells structure or layers composed of quantum dots or quantum dashes, electrically separated from each other.

6. The device according to one of claims 2 to 5 wherein the semiconductor have a GaN substrate and top layer forming a p-n junction, the substrate and the top layer having opposite doping.

7. The device according to one of claims 2 to 6 wherein the semiconductor diode is insulated by an insulating layer.

8. The device according to one of claims 2 to 7 further comprising a first microlens array at an entry of the semiconductor optical amplifiers array and adapted to focus the image of an object on the retina.

9. The device according to claim 8 further comprising a second microlens array at an output of the semiconductors array and adapted to compensate a divergence of the amplified light beam at the output of the semiconductor optical amplifiers array.

10. The device according to one of claims 1 to 9 further comprising an optical coating adapted to suppress the amplified spontaneous emission generated in the optical amplifiers and avoiding the generation of a laser.

11. The device according to claim 10 wherein the optical coating is an antireflective coating.

12. The device according to one of claims 1 to 11 wherein the optical amplifier means are adapted to specifically amplify light having a wavelength comprised within a predetermined range.

13. The device according to claim 12 wherein the optical amplifier means are adapted to stimulate light-sensitive proteins incorporated in the retina area by an optogenetic technique.

14. The device according to claim 10 wherein the optical coating is also adapted to filter the amplifier light at a wavelength range adapted to stimulate light-sensitive proteins incorporated in the retina area by an optogenetic technique.

15. An active crystalline lens prosthesis comprising a device according to one of claims 1 to 14.

16. A corrective glass comprising a device according to one of claims 1 to 14.

17. A method for amplifying the light incoming on a retina comprising:
placing a device according to one of claims 1-16 in front of the retina of a user, so that the light incoming on the retina is amplified by the optical amplifier means; and
adjusting the gain of the optical amplifier means.

18. The method of claim 17, further comprising a step of configuring the device to form a clear image of a distant scene on the retina.
